(19) Europäisches Patentamt
European Patent Office
Office européen des brevets

(11) Veröffentlichungsnummer: **0 023 032**
**A1**

(12) # EUROPÄISCHE PATENTANMELDUNG

(21) Anmeldenummer: **80104171.6**

(22) Anmeldetag: **17.07.80**

(51) Int. Cl.³: **C 07 D 473/06**
C 07 D 473/04, C 07 D 473/08
C 07 D 473/10
//A61K31/52

(30) Priorität: **21.07.79 DE 2929596**

(43) Veröffentlichungstag der Anmeldung:
**28.01.81 Patentblatt 81/4**

(84) Benannte Vertragsstaaten:
**AT CH LI NL SE**

(71) Anmelder: **HOECHST AKTIENGESELLSCHAFT**
**Zentrale Patentabteilung Postfach 80 03 20**
**D-6230 Frankfurt/Main 80(DE)**

(72) Erfinder: **Furrer, Harald, Dr.**
**Breslauer Strasse 27**
**D-6233 Kelkheim (Taunus)(DE)**

(54) **Verfahren zur Herstellung von Oxoalkyl-xanthinen.**

(57) Verfahren zur Herstellung von Verbindungen der allgemeinen Formel I

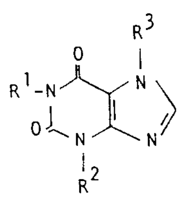
I

in der $R^1$ und $R^3$ einen geradkettigen ($\omega$-1)-Oxoalykylrest mit 4 - 8 Kohlenstoffatomen oder ($C_1$-$C_{12}$)-Alkyl bedeuten, wobei jedoch mindestens einer der Reste $R^1$ oder $R^3$ eine Oxoalkylgruppe darstellt und wobei $R_2$ und $R_3$ nicht gleichzeitig Methyl sind, wenn die Oxoalkylgruppe ein 5-Oxohexylrest ist, bei dem man Salze von 3-Alkylxanthinen, 1,3- bzw. 3,7 Dialkylxanthinen oder von 1-Oxoalkyl-3-alkyl- bzw. 3-Alkyl-7-Oxoalkylxanthinen mit Alkylsulfonyloxoalkan-2-onen oder Arylsulfonyloxyalkan-2-onen der allgemeinen Formel II

O
‖
$CH_3$-CO-$(CH_2)_n$-O-S-R    II
‖
O

in der R einen aliphatischen oder aromatischen Rest bedeutet und n für 2 - 6 steht, umsetzt.

EP 0 023 032 A1

Verfahren zur Herstellung von Oxoalkyl-xanthinen

Die Erfindung betrifft ein Verfahren zur Herstellung von Oxoalkyl-xanthinen, bei dem Xanthine mit Alkylsulfonyloxyalkanonen oder mit Arylsulfonyloxyalkanonen umgesetzt werden.

Es ist bekannt, daß man (5-Oxohexyl)-xanthine aus Xanthinen und 6-Halogenhexan-2-onen erhalten kann. Auch durch Umsetzung von N-(3-Halogenpropyl)-xanthinen mit Natriumacetessigestern, anschließende Verseifung und Decarboxylierung wurden (5-Oxohexyl)-xanthine erhalten.

Gegenstand der Erfindung ist ein Verfahren zur Herstellung von Verbindungen der allgemeinen Formel I

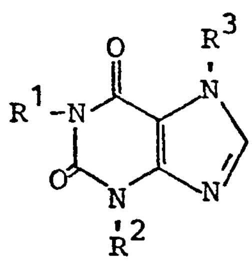

I

in der $R^1$ und $R^3$ einen geradkettigen (w-1)-Oxoalkylrest mit 4 - 8 Kohlenstoffatomen oder $(C_1-C_{12})$-Alkyl und $R^2$ $(C_1-C_8)$-Alkyl bedeuten, wobei jedoch mindestens einer der Reste $R^1$ oder $R^3$ eine Oxoalkylgruppe darstellt und wobei $R_2$ und $R_3$ nicht gleichzeitig Methyl sind, wenn die Oxoalkylgruppe ein 5-Oxohexylrest ist. Das Verfahren ist dadurch gekennzeichnet, daß man zur Einführung des Oxoalkylrestes Salze von 3-Alkylxanthinen, 1,3- bzw. 3,7-Dialkylxanthinen oder von 1-Oxoalkyl-3-alkyl bzw. 3-Alkyl-7-Oxoalkyl-xanthinen mit Alkylsulfonyloxyalkan-2-onen oder Arylsulfonyloxyalkan-2-onen der allgemeinen Formel

$$CH_3-CO-(CH_2)_n-O-\underset{\underset{O}{\parallel}}{\overset{\overset{O}{\parallel}}{S}}-R \qquad II$$

umsetzt, wobei R einen aliphatischen Rest, vorzugsweise einen Alkylrest mit 1 - 4 C-Atomen, wie $CH_3$-, $C_2H_5$-, $CF_3$- oder einen aromatischen Rest, wie Phenyl, p-Tolyl, p-Bromphenyl, vorzugsweise aber Methyl und p-Tolyl darstellt und n 2 bis 6 und vorzugsweise 4 ist. Die einzelnen Ausgangsstoffe können in stöchiometrischen oder auch in nicht-stöchiometrischen Mengen verwendet werden. Man kann die Salze der Xanthine, vorzugsweise Alkalisalze, in fertiger Form einsetzen. Vorteilhaft erzeugt man diese Salze aber im Reaktionsgemisch, vorzugsweise mit Kalium-carbonat.

Das erfindungsgemäße Verfahren läßt sich in technisch einfacher Weise im allgemeinen bei einer Temperatur von 20 bis 160°C, vorzugsweise von 60 bis 140°C, gegebenenfalls bei erhöhtem oder vermindertem Druck, aber gewöhnlich bei Atmosphärendruck durchführen. Als Lösungs- oder Verteilungsmittel eignen sich unter den Reaktionsbedingungen inerte Verbindungen, wie z.B. Dimethylformamid, Dimethylsulfoxid, Aceton, Butan-2-on oder Acetonitril. Die Alkylsulfonyloxyalkan-2-one und Arylsulfonyloxyalkan-2-one der Formel II lassen sich nach bekannten Methoden aus Hydroxyalkan-2-onen und Halogeniden der entsprechenden Sulfonsäuren erhalten (vgl. z.B. Organikum, VEB Deutscher Verlag der Wissenschaften, Berlin 1976, S. 683 ff.).

Die erfindungsgemäß erhaltenen (5-Oxohexyl)-xanthine sind bekannt und wirken fördernd auf die cerebrale und periphere Durchblutung und/oder bronchospasmolytisch und/oder fibrinolytisch.

Beispiele:

1. 1-(5-Oxohexyl)-3-methyl-7-propylxanthin

a) Zu 34,9 g 6-Hydroxyhexan-2-on und 36,4 g Triäthylamin in 500 ml Methylenchlorid tropft man unter Rühren bei 0 - 5°C Innentemperatur 36,1 g Methansulfonsäurechlorid. Es wird 1 Stunde bei Raumtemperatur nachgerührt,

der Niederschlag abfiltriert und das Filtrat mit $NaHCO_3$-Lösung und Wasser neutral und salzfrei gewaschen. Nach Trocknen über $Na_2SO_4$ entfernt man das Methylenchlorid bei vermindertem Druck und erhält 57,4 g 6-Mesyloxyhexan-2-on, das dünnschicht-chromatographisch einheitlich ist (Kieselgel, Laufmittel: Diäthyläther, Entwicklung mit Eisessig/$H_2SO_4$, Volumenverhältnis 1:1). Das Produkt konnte direkt zur Einführung des (5-Oxohexyl)-Restes in Xanthine eingesetzt werden.

<u>IR-Spektrum (kap.):</u>

1710 $cm^{-1}$ (C=O), 1360 $cm^{-1}$, 1180 $cm^{-1}$ (Mesylat)

<u>$^1H$-Kernresonanzspektrum (in $CDCl_3$):</u>

$\delta$(ppm) = 4 - 4,3 (m, $-CH_2-O-\overset{O}{\underset{O}{S}}-$), 2,95 (s, $-O-\overset{O}{\underset{O}{S}}-CH_3$),

2,2-2,6 (m, fast t, $-CH_2-\underset{O}{C}-$), 2,1 (s, $-\underset{O}{C}-CH_3$), 1,1-1,9

(m, $-(CH_2)_2-$)

b) Das Gemisch aus 10,4 g 3-Methyl-7-propyl-xanthin und 7,1 g $K_2CO_3$ in 175 ml Dimethylformamid wird unter Rühren auf 120°C erhitzt. Während 1/2 Stunde tropft man 10 g 6-Mesyloxyhexan-2-on in 20 ml Dimethylformamid zu und rührt weitere 7 Stunden bei 120°C. Nach Einengen des Ansatzes bei vermindertem Druck wird der Rückstand mit 1 n Natronlauge auf pH 13 - 14 gestellt und mehrmals mit Methylenchlorid extrahiert. Die gesammelten Methylenchloridextrakte werden bei vermindertem Druck eingeengt und der Rückstand 1 Stunde in 50 ml 1 n Salzsäure bei Rückflußtemperatur erhitzt. Nach dem Abkühlen stellt man mit 1 n Natronlauge auf pH 13 - 14, extrahiert mehrmals mit Methylenchlorid, wäscht die gesammelten Methylenchloridphasen neutral, trocknet und engt ein. Aus dem Rückstand werden nach Reinigung durch Umkristallisation aus Isopropanol/Petroläther (Volumenverhältnis 1:2) 11,5 g

1-(5-Oxohexyl)-3-methyl-7-propylxanthin vom Schmelzpunkt 70 - 71°C erhalten. Das Verfahrensprodukt ist gemäß Mischschmelzpunkt, Dünnschichtchromatogramm, Infrarot- und Kernresonanzspektren identisch mit der authentischen Substanz.

2. 1,3-Dimethyl-7-(5-oxohexyl)xanthin

a) 6-Tosyloxyhexan-2-on

Zu 23,5 g 6-Hydroxyhexan-2-on in 200 ml absolutem Pyridin gibt man bei -5°C auf einmal 38,1g p-Toluolsulfochlorid hinzu, rührt 2 1/2 Stunden bei 0 bis -5°C nach und tropft innerhalb einer halben Stunde bei max. 0°C 20 ml $H_2O$ zu. Nach Versetzen mit 500 g Eis wird mit 3 x 250 ml Methylenchlorid ausgeschüttelt. Die gesammelten Methylenchloridextrakte werden mit 2 n Salzsäure pyridinfrei, mit gesättigter $NaHCO_3$-Lösung und $H_2O$ neutral gewaschen, über $Na_2SO_4$ getrocknet und bei vermindertem Druck eingeengt. Man erhält 40,8 g 6-Tosyloxyhexan-2-on, das direkt zur Einführung des (5-Oxohexyl)-Restes in Xanthine eingesetzt werden konnte.

IR-Spektrum (kap.):

1710 $cm^{-1}$ (C=O), 1360 $cm^{-1}$, 1190$cm^{-1}$, 1180 $cm^{-1}$ (Tosylat)

$^1$H-Kernresonanzspektrum (in $CDCl_3$):

$\delta$(ppm)=7,4; 7,82 (d,d, 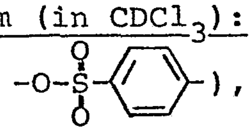), 3,85 - 4,2 (m, $-CH_2-O-\overset{O}{\underset{O}{S}}-$), 2,2 - 2,7 (s + m, $CH_3$-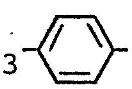, $-CH_2-\underset{O}{C}-$), 2,1 (s, $-\underset{O}{C}-CH_3$), 1,3 - 1,9 (m, $-(CH_2)_2-$)

b) Zu der Lösung von 9 g Theophyllin in 175 ml Dimethylformamid werden bei 120°C unter Rühren 7,1 g $K_2CO_3$ zugegeben und innerhalb einer 1/2 Stunde 14,2 g 6-Tosyloxyhexan-2-on in 20 ml Dimethylformamid zuge-

0023032

tropft. Nach weiteren 5 Stunden Rühren bei 120°C engt man bei vermindertem Druck ein, versetzt den Rückstand mit 55 ml 1 n Natronlauge und extrahiert mit Methylenchlorid. Die gesammelten Methylenchloridphasen werden mit 30 ml 1 n Natronlauge behandelt, mit $H_2O$ neutral gewaschen, über $Na_2SO_4$ getrocknet und eingeengt. Aus dem Rückstand erhält man nach Reinigung durch Umkristallisation aus Isopropanol/Petroläther (Volumenverhältnis 1:2) 9,3 g 1,3-Dimethyl-7-(5-oxohexyl)-xanthin vom Schmelzpunkt 76 - 77°C.

Das Verfahrensprodukt ist gemäß Mischschmelzpunkt, Dünnschichtchromatogramm, Infrarotspektrum und Kernresonanzspektrum identisch mit der authentischen Substanz.

Nach der Arbeitsweise der obigen Beispiele werden folgende (5-Oxohexyl)-xanthine hergestellt und in gleicher Weise wie dort identifiziert.

3. 1-Propyl-3-methyl-7-(5-oxohexyl)-xanthin, Schmelzpunkt: 76 - 78°

4. 1-Hexyl-3-methyl-7-(5-oxohexyl)-xanthin, Schmelzpunkt: 35 - 38°

5. 1-(5-Oxohexyl)-3-methyl-7-äthylxanthin, Schmelzpunkt: 102 - 103°

6. 1-(5-Oxohexyl)-3-methyl-7-butylxanthin, Schmelzpunkt: 79 - 80°

7. 1-(5-Oxohexyl)-3-methyl-7-hexylxanthin, Schmelzpunkt: 52°C

8. 1-(5-Oxohexyl)-3-methyl-7-decyl-xanthin, Schmelzpunkt: 64 - 66 °

9. 1-(5-Oxohexyl)-3-äthyl-7-propyl-xanthin, Schmelzpunkt: 81 - 82°

10. 1-Methyl-3-butyl-7-(5-oxohexyl)-xanthin, $n_D^{20}$ 1,5308.

Patentansprüche:

1. Verfahren zur Herstellung von Verbindungen der allgemeinen Formel I

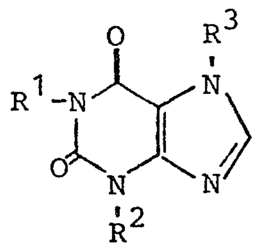

I

in der $R^1$ und $R^3$ einen geradkettigen (w-1)-Oxoalkylrest mit 4 - 8 Kohlenstoffatomen oder $(C_1-C_{12})$-Alkyl und $R^2$ $(C_1-C_8)$-Alkyl bedeuten, wobei jedoch mindestens einer der Reste $R^1$ oder $R^3$ eine Oxoalkylgruppe darstellt und wobei $R_2$ und $R_3$ nicht gleichzeitig Methyl sind, wenn die Oxoalkylgruppe ein 5-Oxohexylrest ist, dadurch gekennzeichnet, daß man Salze von 3-Alkylxanthinen, 1,3- bzw. 3,7-Dialkylxanthinen oder von 1-Oxoalkyl-3-alkyl- bzw. 3-Alkyl-7-Oxoalkylxanthinen mit Alkylsulfonyloxyalkan-2-onen oder Arylsulfonyloxyalkan-2-onen der allgemeinen Formel II

$$CH_3-CO-(CH_2)_n-O-\underset{\underset{O}{\|}}{\overset{\overset{O}{\|}}{S}}-R \qquad II$$

in der R einen aliphatischen oder aromatischen Rest bedeutet und n für 2 - 6 steht, umsetzt.

2. Verfahren nach Anspruch 1, dadurch gekennzeichnet, daß in Formel II R für einen Alkylrest mit 1 - 4 C-Atomen, vorzugsweise Methyl, Äthyl oder Trifluormethyl steht.

3. Verfahren nach Anspruch 1, dadurch gekennzeichnet, daß in Formel II R für Phenyl, p-Tolyl oder p-Bromphenyl steht.

4. Verfahren nach einem der Ansprüche 1 - 3, dadurch gekennzeichnet, daß in Formel II n für 4 steht.

5. Verfahren nach einem der Ansprüche 1 - 4, dadurch gekennzeichnet, daß die Xanthine in Form ihrer Alkalisalze eingesetzt werden.

6. Verfahren nach einem der Ansprüche 1 - 5, dadurch gekennzeichnet, daß sowohl die Salzbildung der Xanthine in situ mit $K_2CO_3$ als auch die Umsetzung der Xanthinsalze in einem unter den Reaktionsbedingungen gegenüber den Reaktionspartnern inerten Lösungs- oder Verteilungsmittel erfolgt.

7. Verfahren nach einem der Ansprüche 1 - 5, dadurch gekennzeichnet, daß die Umsetzungen bei einer Temperatur von 20 bis 160°C, vorzugsweise von 60 bis 140°C, durchgeführt werden.

0023032

Nummer der Anmeldung

Europäisches Patentamt

# EUROPÄISCHER RECHERCHENBERICHT

EP 80104171.6

| EINSCHLÄGIGE DOKUMENTE | | | KLASSIFIKATION DER ANMELDUNG (Int Cl ³) |
|---|---|---|---|
| Kategorie | Kennzeichnung des Dokuments mit Angabe, soweit erforderlich, der maßgeblichen Teile | betrifft Anspruch | |
| | DE - A1 - 2 716 210 (DEGUSSA)<br>+ Anspruch 28c; Seiten 28, 29 +<br>-- | 1,5,7 | C 07 D 473/06<br>C 07 D 473/04<br>C 07 D 473/08<br>C 07 D 473/10//<br>A 61 K 31/52 |
| | FR - A1 - 2 336 403 (LABOR-BRUNEAU)<br>+ Seiten 1,2 +<br>-- | 1,5,7 | |
| | DE - A1 - 2 714 953 (HOECHST)<br>+ Anspruch 8; Seiten 5,6 +<br>---- | 1,5-7 | |
| | | | RECHERCHIERTE SACHGEBIETE (Int. Cl.³) |
| | | | C 07 D 473/00 |
| | | | KATEGORIE DER GENANNTEN DOKUMENTE<br>X: von besonderer Bedeutung<br>A: technologischer Hintergrund<br>O: nichtschriftliche Offenbarung<br>P: Zwischenliteratur<br>T: der Erfindung zugrunde liegende Theorien oder Grundsätze<br>E: kollidierende Anmeldung<br>D: in der Anmeldung angeführtes Dokument<br>L: aus andern Gründen angeführtes Dokument<br>&: Mitglied der gleichen Patentfamilie, übereinstimmendes Dokument |
| X | Der vorliegende Recherchenbericht wurde für alle Patentansprüche erstellt. | | |

| Recherchenort | Abschlußdatum der Recherche | Prüfer |
|---|---|---|
| WIEN | 30-09-1980 | DASCHL |